# EUROPÄISCHE PATENTSCHRIFT

(11) **EP 2 400 858 B1**
(45) Veröffentlichungstag und Bekanntmachung des Hinweises auf die Patenterteilung: **07.04.2021**
(21) Anmeldenummer: 10707796.8
(22) Anmeldetag: 26.02.2010
(51) Int. Cl.: A23J 1/14, A61K 8/64, A61Q 19/00, A23K 10/30, A23K 20/147, A23K 50/80, A23L 33/185

(54) **AUS RAPSSAMEN HERGESTELLTES PROTEINPRÄPARAT**
PROTEIN PREPARATION PRODUCED FROM RAPE SEEDS
PRÉPARATION PROTÉIQUE À BASE DE GRAINES DE COLZA

(30) Priorität: 27.02.2009 DE 102009010813
(43) Veröffentlichungstag der Anmeldung: 04.01.2012
(73) Patentinhaber: Fraunhofer-Gesellschaft zur Förderung der angewandten Forschung e.V., 80686 München (DE)
(72) Erfinder: PICKARDT, Claudia, 12203 Berlin (DE); EISNER, Peter, 85354 Freising (DE); BADER, Stephanie, 85356 Freising (DE); MÜLLER, Klaus, 85354 Freising (DE); ZIMMERMANN, Hubert, CH-8932 Mettmenstetten (CH); BERNATH, Etienne, CH-8803 Rüschlikon (CH); WILD, Florian, 85354 Freising (DE); FRANKL, Michael, 81241 München (DE); GRUPPE, Sigrid, 85391 Allershausen (DE); SCHREIBER, Klaus, 85354 Freising (DE)
(74) Vertreter: AMMANN PATENTANWÄLTE AG
(86) Internationale Anmeldenummer: PCT/CH2010/000047
(87) Internationale Veröffentlichungsnummer: WO 2010/096943

(56) Entgegenhaltungen:
- WO-A1-2007/033481
- WO-A1-2009/137934
- WO-A2-2004/000032
- DE-A1- 2 421 270
- DE-A1- 19 912 037
- UA-U- 25 695
- US-A- 4 158 656
- US-A- 4 219 469
- US-A- 4 244 973
- US-A1- 2007 178 566
- Jens-Peter Dr. Krause ET AL: "Rapsprotein in der Humanernährung", UFOP-Schriften, Heft 32, 1 January 2007 (2007-01-01), pages 1-105, XP055525677, Berlin Retrieved from the Internet: URL:https://www.ufop.de/files/4813/3922/62 80/UFOP_Schriften_Heft_32.pdf [retrieved on 2018-11-21]

## Beschreibung

Die vorliegende Erfindung bezieht sich auf ein aus Rapssamen hergestelltes Proteinpräparat und auf ein Verfahren zur Herstellung eines derartigen Proteinpräparats.

Proteinpräparate finden vielfach Anwendung in Lebensmitteln als ernährungsphysiologisch oder technofunktionell aktive Zutaten. Es gibt Proteinpräparate mit besonders hoher Proteinwertigkeit zur Anwendung als hochwertige Nahrungsmittelzusätze (Babynahrung, Spezialernährung, Sportlernahrung). Diese sind prinzipiell auch für die Formulierung von Futtermitteln von Interesse, in denen eine hohe Proteinverfügbarkeit gewährleistet werden muss. Andere Proteinpräparate haben eine gute Technofunktionalität und sind z. B. dafür geeignet, Schäume oder Emulsionen zu stabilisieren oder Gele auszubilden. Diese Proteinpräparate sind vorrangig als Lebensmittelzutaten geeignet und finden ausserdem Anwendung für Spezialfuttermittel oder technische Zwecke.

Grundsätzlich können Proteinpräparate tierischen und pflanzlichen Ursprungs unterschieden werden.

Beispiele von Proteinpräparaten tierischen Ursprungs sind solche aus Hühnerei, Milch, Molke oder Kasein und Gelatinepräparate aus Schlachtabfällen. Nachteilig ist, dass solche Proteinpräparate einen charakteristischen Eigengeschmack und Eigengeruch aufweisen und daher auf bestimmte Anwendungen beschränkt sind. Oft sind sie teuer in der Herstellung und problematisch in Bezug auf Allergien und werden von bestimmten Verbrauchern aus ethischen Gründen abgelehnt.

Als alternative Proteinquellen pflanzlicher Herkunft werden vor allem Präparate aus Sojaproteinen und Weizenkleber verwendet. Ihr Anwendungsspektrum bei Lebens- und Futtermitteln ist jedoch eingeschränkt u. a. aufgrund geruchsaktiver, geschmacksaktiver und/oder allergieauslösender Substanzen und/oder dunkler Farbe.

Bekannt sind ebenfalls pflanzliche Proteinpräparate, welche aus Rapssamen hergestellt werden in Form von Rapsproteinkonzentraten oder Rapsproteinisolaten.

Im Vergleich zu Rapsproteinkonzentraten haben Rapsproteinisolate einen sehr hohen Proteingehalt, der bezogen auf die Trockenmasse mindestens 90 % beträgt. Rapsproteinkonzentrate enthalten jedoch in der Regel das gesamte Aminosäurespektrum des bei der Herstellung verwendeten Rapssamens. Sie haben ein ausgewogenes Aminosäurespektrum und eignen sich u. a. zur Proteinanreicherung in Lebens- und Futtermitteln.

Die Herstellung von Rapsproteinkonzentraten erfordert besondere Massnahmen, wenn die für spezielle Anwendungen unerwünschten Komponenten wie Glucosinolate, Phenolsäuren und Phytinsäure beseitigt werden sollen. Zu diesem Zweck wurde eine Extraktion mit verdünnten wässrig-alkoholischen Lösungen vorgeschlagen. Beispielsweise werden in der Literatur Rapsproteinkonzentrate beschrieben, welche durch Extraktion mit 45 %igem Isopropanol gewonnen wurden (siehe Yumiko Yoshie-Stark et al., "Functional and bioactive properties of rapeseed protein concentrates and sensory analysis of food application with rapeseed protein concentrates", LWT 39 (2006) 503-512) oder mit 80 %igem Alkohol in Verbindung mit hohen Temperaturen von 60°C (siehe Kozlowska et al., "The influence of selected technological processes on the improvement of rapeseed meal and fluor feed quality. Part 1.", Die Nahrung 35 (1991) 5, 485-489).

Bei diesen beiden Verfahren werden Rapsproteinkonzentrate hergestellt, indem vorgängig zur Extraktion ausschliesslich mittels Lösemittel entölt wird. Nach der Extraktion der proteinfremden Stoffe sind keine weiteren Behandlungen vorgesehen. Die Verfahren haben u. a. den Nachteil, dass die funktionellen Eigenschaften der Rapsproteinkonzentrate schlecht vorgebbar sind. Auch wird im oben erwähnten Zeitschriftenartikel von Yumiko Yoshie-Stark et al. berichtet, dass Wurstwaren, bei denen das hergestellte Rapsproteinkonzentrat verwendet wurde, eine starke Farbverschlechterung aufwiesen.

Nebst der Extraktion kann auch bereits die Aufbereitung der Rapssamen problematisch sein, wenn diese nicht schonend genug erfolgt. Im Zeitschriftenartikel von Felix H. Schneider und Michael Rass, "Trennpressen geschälter Rapssaat - Zielsetzung und verfahrenstechnische Probleme", Fett/Lipid 99 (1997) Nr. 3, 91-98, wird ein Verfahren beschrieben, bei welchem geschälte Rapssamen mittels Schneckenpresse und anschliessend mittels Hexan bei einer Temperatur von 60 Grad Celsius entölt werden. Es sind keine speziellen Massnahmen für eine schonende Entölung vorgesehen, sodass die Proteine übermässig denaturiert sein können.

Insgesamt sind die bekannten Rapsproteinkonzentrate, welche einen niedrigen Aufreinigungsgrad aufweisen, eingeschränkt in ihrer Funktionalität und/oder enthalten einen gewissen Anteil störender Komponenten, die den Nährwert, die Farbe, den Geruch und/oder Geschmack der sie enthaltenden Lebens- oder Nahrungsmittel sehr negativ beeinflussen können. Die Rapsproteinkonzentrate haben daher eine beschränkte Applikationsbreite und sind nur in niedrigen Konzentrationen einsetzbar.

Rapsproteinisolate, wie sie z. B. in **der** EP 1 513 415 B1 beschrieben sind, weisen einen hohen Aufreinigungsgrad auf und sind daher aufwändig und teuer in der Herstellung. Sie enthalten nur einen Anteil an Einzelkomponenten der ursprünglichen Proteinmatrix des Rapssamens und haben deswegen ein spezifisches ernährungsphysiologisches und technofunktionelles Profil mit wenigen besonders hervorgehobenen Eigenschaften. Ihr Anwendungsspektrum ist sehr spezifisch und auf wenige Anwendungen begrenzt.

Ein Verfahren zur Zubereitung eines Rapsproteinkonzentrates für den menschlichen Verbrauch, welches nicht toxisch ist, eine angenehme helle Farbe und einen neutralen angenehmen Geschmack hat, wird weiterhin in DE 24 21 270 A1 beschrieben. Das Verfahren umfasst das Laugen des hülsenfreien Fleischmaterials, Trocknen, Vorpressen, Lösungsmittelextrahieren und gegebenenfalls Mahlen.

Eine Aufgabe der vorliegenden Erfindung ist es, Proteinpräparate zu schaffen, die relativ kostengünstig herstellbar und vielseitig einsetzbar sind.

Diese Aufgabe wird gelöst mit einem Proteinpräparat, das im Anspruch 1 definiert oder mit dem Verfahren gemäss dem unabhängigen Verfahrensanspruch hergestellt ist. Die weiteren Ansprüche geben bevorzugte Ausführungsformen des erfindungsgemässen Proteinpräparats und Verfahrens an, sowie eine Verwendung eines erfindungsgemässen Proteinpräparats.

Das erfindungsgemässe Proteinpräparat weist einen tieferen Proteingehalt auf als Proteinisolate und ist für eine kostengünstigere Herstellung zugänglich, da eine hohe Aufreinigung, wie sie bei den Proteinisolaten erforderlich ist, vermieden werden kann.

Überraschenderweise weist das erfindungsgemässe Proteinpräparat trotz des höheren Anteils an proteinfremden Stoffen Eigenschaften auf, die ähnlich wie die bekannten Rapsproteinisolate oder sogar vielfältiger als diese sind. Aufgrund der hellen Farbe sowie des ausgewogenen technofunktionellen Spektrums in Form der wasserbindenden, ölbindenden sowie emulgierenden Funktionalität ist das erfindungsgemässe Proteinpräparat vielseitig einsetzbar, u. a. in Lebens- und Futtermitteln, um Wasser und/oder Öl zu binden und/oder eine Emulsion zu bilden. Das Proteinpräparat ist geeignet andere Präparate zu ersetzen, welche für diese Funktionalitäten bis anhin verwendet wurden und welche tierischen oder pflanzlichen Ursprungs sind wie Hühnerei, Milch, Soja, Raps in Form von Rapsisolaten, etc.

Weiter überraschenderweise weist das Proteinpräparat, das in Form des besonders kostengünstig herstellbaren Rapsproteinmehls vorliegt, Eigenschaften hinsichtlich Farbe und Funktionaliäten auf, die es erlauben, das Rapsproteinmehl bei zahlreichen Lebensmittel- und Futtermittelanwendungen einzusetzen.

Der Anwendungsbereich des erfindungsgemässen Proteinpräparats kann noch weiter erstreckt werden, wenn das Proteinpräparat frei von den pflanzen- oder saateigenen Aromen des Rapses ist, insbesondere wenn es im Wesentlichen geruchsfrei und/oder im Wesentlichen geschmacksneutral ist. Dadurch wird u. a. vermieden, dass es bei der Einarbeitung des Proteinpräparats in Lebens- und Futtermitteln zu einer unerwünschten Geschmacks- und Aromaänderung kommt.

Auch durch das Erhalten einer schaumbildenden Funktionalität kann der Anwendungsbereich erweitert werden, so dass das Proteinpräparat z. B. als Ersatz für Hühnereiklar oder andere schaumbildende Zusätze verwendet werden kann, um schaumartige Lebensmittel herzustellen.

Vorzugsweise weist das Proteinpräparat einen tiefen Fettgehalt auf, wodurch eine gute Lagerstabilität des Proteinpräparats gewährleistet wird.

Weiter vorzugsweise weist das Proteinpräparat einen tiefen Gehalt an Phytinsäure, Glucosinolaten und/oder Phenolsäure auf. Dadurch wird u. a. die Gefahr vermindert, dass bei der Verdauung die Verwertung von Nährstoffen beeinträchtigt ist.

Das erfindungsgemässe Verfahren erlaubt eine schonende Herstellung des Präparates, so dass einer unerwünschten Denaturierung der Proteine vorgebeugt wird, und ermöglicht es, qualitativ und sensorisch hochwertige Proteinpräparate mit einem breiten Applikationsspektrum zu gewinnen.

Durch die bei der mechanischen Entölung vorgesehene Teilentölung, ist die Behandlung besonders schonend und es können die anschliessenden Verfahrensschritte vereinfacht werden, indem z. B. eine mechanische Zerkleinerung vor der Extraktion entfällt.

Wird bei der mechanischen Entölung eine zu hohe Temperatur vermieden, sind in besonderem Masse unerwünschte Proteinveränderungen oder Reaktionen der proteinfremden Stoffe mit den Proteinen vermeidbar.

Bei der Herstellung eines Proteinkonzentrates ist eine schonende Behandlung auch dadurch erzielbar, indem eine Extraktion vorgesehen ist, in welcher die proteinfremden Stoffe aus dem Proteinmehl abgereichert werden. Durch eine nachfolgende Aufbereitung in der Korngrösse sind die funktionellen Eigenschaften des Proteinkonzentrates in verbessertem Masse vorgebbar.

Vorzugsweise wird das Verfahren so durchgeführt, dass die Extraktion mittels des Extraktionslösungsmittels in mehreren Extraktionsschritten durchgeführt wird, wobei bei mindestens einem Übergang von dem einem zum nächsten Extraktionsschritt der Alkoholgehalt im Extraktionslösungsmittel erhöht wird. Dies erlaubt es, die anschliessende Trocknung besonders schonend zu gestalten, da der Anteil des zu entfernenden Restwassers, das weniger schnell und bei einer höheren Temperatur als der Alkohol verdampft, reduziert ist.

Weitere Vorteile ergeben sich aus den Merkmalen gemäss den abhängigen Ansprüchen sowie der nachfolgenden Beschreibung, in welcher die Erfindung anhand von Ausführungsbeispielen erläutert wird.

Soweit nicht anders vermerkt, beziehen sich die nachfolgend in Prozent angegebenen Gehaltsangaben bei Flüssigkeiten auf Volumenprozent (v/v) bei einer Temperatur von 25 Grad Celsius und bei Feststoffen auf Masseprozent (w/w).

### A) Herstellungsverfahren

Das erfindungsgemässe Proteinpräparat kann beispielsweise nach folgendem Verfahren aus Raps hergestellt werden. Dabei kann ein beliebiger Raps genommen werden, auch ein speziell gezüchteter (z. B. die Sorte "00-Raps", im Englischen "Canola") und/oder ein gentechnisch veränderter. Das Verfahren umfasst folgende Verfahrensschritte V1-V8:
V1 Vorbehandlung:
   Die Rapssaat wird nach der Ernte bei einer Produkttemperatur von unter 95°C, bevorzugt unter 40°C bei einer Heisshaltezeit getrocknet, die typischerweise im Bereich von 10 - 20 min liegt. Eine höhere Produkttemperatur ist denkbar, wenn die Kontaktzeit kurz ist, d. h. weniger als 20 min und vorzugsweise weniger als 5 min. Durch die Trocknung wird erreicht, dass die Rapssaat einen Wassergehalt von weniger als 9 % w/w aufweist, bevorzugt weniger als 8 % w/w und besonders bevorzugt weniger als 7 % w/w. Im Weiteren wird die Trocknung gezielt so durchgeführt, dass einerseits Enzyme inaktiviert werden, die nachfolgend das Verfahren oder die Qualität des Endprodukts stören würden, und andererseits in begrenztem Umfang eine Denaturierung des Speicherproteins stattfindet, das den Hauptanteil der Proteine ausmacht. So kann durch die Inaktivierung der Enzyme wie Myrosinase, Lipoxigenase und Polyphenoloxidase u. a. verhindert werden, dass es nachfolgend zu einer Freisetzung von geruchs- und geschmacksaktiven Senfölen kommt, zu einer Fettspaltung und/oder zu Farbveränderungen.
V2 Schälung:
   Die Rapssamen, welche aus Kern und Schalen bestehen, werden durch Aufschluss in einer Mühle und Auftrennung in eine kernreiche und eine schalenreiche Fraktion im Luftstrom geschält. Der Schälprozess ist so gestaltet, dass die im nachfolgenden Verfahrensschritt V3 verwendete Kernfraktion einen Schalenanteil von weniger als 5 % w/w und besonders bevorzugt weniger als 1 % w/w aufweist. Gegebenenfalls umfasst der Schälprozess zusätzlich ein Sieben der Rapssamen oder der Fraktionen und/oder ein weiteres Sichten der Fraktionen, um eine genügend aufgereinigte Kernfraktion zu erhalten.
V3 Mechanische Behandlung:
   Die Kernfraktion wird mechanisch, mittels Presse, teilentölt bis zu einem Restölgehalt von 15-35 % w/w, vorzugsweise 15-25 % w/w. Die über die Zeitdauer des Pressvorganges gemittelte Temperatur Tm der Kernfraktion liegt dabei unter 60°C. Dabei ist nicht ausgeschlossen, dass die Temperatur der Kernfraktion zu einem bestimmten Zeitpunkt und/oder die lokale Temperatur bei einzelnen Kernen höher als Tm sind. Gegebenenfalls ist eine Kühlung vorgesehen, um die Wärme abzuführen, welche bei der mechanischen Entölung z. B. aufgrund des Druckes beim Pressen entsteht. Es ist denkbar, ergänzend zur mechanischen Entölung eine mechanische Behandlung in Form einer Flockierung ("flaking") vorzusehen.
V4 Lösemittel-Entölung:
   Der im Verfahrensschritt V3 erhaltene Pressrückstand ("Rapspresskuchen") wird mit n-Hexan, iso-Hexan oder einem anderen geeigneten Lösemittel bei Temperaturen unter 90°C, vorzugsweise unter 60°C entölt und das Lösemittel entfernt, z. B. mittels Flash-Desolventierung. Der entölte Rückstand weist bezogen auf die Trockenmasse einen Restgehalt an Öl von weniger als 6 % (bestimmt nach der Caviezel-Methode) auf und wird nachfolgend auch Rapsproteinmehl genannt.
V5 Wässrig-alkoholische Extraktion:
   Der entölte Rückstand wird mit einem wässrigen Alkohol behandelt, welcher einen Alkoholanteil zwischen 60 und 95 % v/v aufweist, vorzugsweise zwischen 70 und 80 % v/v. Bei der wässrig-alkoholischen Extraktion werden die Proteinbegleitstoffe, wie Zucker, sekundäre Pflanzenstoffe, etc., abgereichert, während die Proteine weitgehend nicht in Lösung gehen. (Dieses Verfahren unterscheidet sich demnach von der Herstellung von Rapsproteinisolaten, bei welcher die Proteine aus der Fasermatrix herausgelöst und anschliessend aus der Lösung zurückgewonnen werden, z. B. durch Fällung und/oder ein Membranverfahren.) Anschliessend an die Extraktion wird der Alkohol entfernt, vorzugsweise unter Einsatz von Vakuum bei Temperaturen von maximal 60°C und/oder unter Verwendung von Alkohol- oder Wasser-Dampf zum Austreiben des Alkohols bei einer Temperatur von unter 60°C.
V6 Trocknung:
   Das im Verfahrensschritt V5 erhaltene Proteinkonzentrat wird getrocknet. Dabei wird die Temperatur u. a. in Abhängigkeit vom Restwassergehalt gewählt und liegt typischerweise im Bereich von 50 bis 90°C, bevorzugt unter 70°C und besonders bevorzugt unter 60°C. Optional wird unter Einsatz von Vakuum getrocknet.
V7 Optionale mechanische Trennung:
   Gegebenenfalls wird das im Verfahrensschritt V6 erhaltene Proteinkonzentrat gesichtet und/oder gesiebt, um die funktionellen Eigenschaften und/oder die Farbe zu modifizieren und um etwaig vorhandene Schalenpartikel abzutrennen.
V8 Weitere Aufbereitung in der Korngrösse:
   Das erhaltene Proteinkonzentrat wird so in eine schüttfähige Form gebracht, dass es eine bestimmte Korngrösse aufweist. Dies geschieht z. B. durch Sieben, Sichten, Feinvermahlung oder Granulierung des im Verfahrensschritt V6 oder V7 erhaltenen Pulvers. Das schliesslich erhaltene Rapsproteinkonzentrat wird nachfolgend auch mit **PRK** bezeichnet.

Es ist es möglich, den Verfahrensschritt V5 wegzulassen und den entölten Rückstand, welchen man im Verfahrensschritt V4 erhält, gemäss den Verfahrensschritten V6, V7 und/oder V8 aufzubereiten. Dadurch wird ein Proteinpräparat in Form eines Rapsproteinmehls (**RPM**) bereitgestellt.

Es wurde festgestellt, dass aufgrund der Aufbereitung in der Korngrösse gemäss dem obigen Verfahrensschritt V7 bzw. V8 eine weitere Verbesserung in den Eigenschaften des Proteinpräparates und/oder eine gezielte Einstellung der funktionellen Eigenschaften erzielt werden kann. Insbesondere wurde festgestellt, dass sich die Wasser- und Ölbindeeigenschaften sowie die Emulgiereigenschaften in unterschiedlicher Weise durch eine Feinvermahlung des Rapsproteinmehls oder Rapsproteinkonzentrats verändern. So können je nach Anwendungszweck eine höhere Wasser- und Ölbindefähigkeit durch gezielte Vermahlung hergestellt und weitere Eigenschaften wie die Emulgierkapazität verändert werden.

### B) Charakterisierung des hergestellten Proteinpräparates

Durch das oben beschriebene Herstellungsverfahren kann ein Proteinpräparat gewonnen werden, das sich z. B. gegenüber Proteinisolaten, die durch wässrige Fraktionierung und aufwändige Isolierungsverfahren gewonnen werden, durch ein ausgewogenes Nährwertprofil und technofunktionelles Spektrum auszeichnen. Das Proteinpräparat ist auch ohne weitere Aufbereitung, um z. B. den hohen Proteingehalt eines Proteinisolates zu erhalten, u. a. als Lebens- oder Futtermittelzutat geeignet. Überraschenderweise zeigt das Proteinpräparat, obwohl es kein Proteinisolat ist, technofunktionelle Eigenschaften von Proteinisolaten auf. Es hat eine neutrale, typischerweise gelbe Farbe und ist weitgehend frei von sensorisch störenden und antinutritiven Begleitstoffen. Insbesondere weist es in Form des Rapsproteinkonzentrates nahezu keinen Eigengeruch und Eigengeschmack auf.

Besonders überraschend ist, dass bereits das entölte Rapsproteinmehl (RPM) eine überaus ansprechende Farbe und sehr ausgeprägte Funktionalität hat und für zahlreiche Lebensmittel- und Futtermittelanwendungen geeignet ist.

Nachfolgend wird zur quantitativen Charakterisierung der hergestellten Proteinpräparate auf folgende Bestimmungsverfahren zurückgegriffen:
- Proteingehalt:
   Der Proteingehalt ist definiert als der Gehalt, der sich aus der Bestimmung des Stickstoff und dessen Multiplikation mit dem Faktor 6,25 errechnet. Der Proteingehalt ist z. B. in Prozent bezogen auf die Trockenmasse (TS) angebbar.
- Farbe:
   Die wahrnehmbare Farbe ist mittels **CIE-L*a*b*-Farbmessung** definiert (vgl. DIN 6417). Dabei gibt die L*-Achse die Helligkeit an, wobei Schwarz den Wert 0 und Weiss den Wert 100 hat, die a*-Achse beschreibt den Grün- oder Rotanteil und die b*-Achse den Blau- oder Gelbanteil.
- Proteinlöslichkeit:
   Die Proteinlöslichkeit ist mittels Bestimmungsverfahren nach Morr et al. 1985 bestimmt (nachfolgend **PN-Bestimmungsverfahren** genannt), siehe den Zeitschriftenartikel: Morr C.V., German, B., Kinsella, J.E., Regenstein, J.M., Van Buren, J.P., Kilara, A., Lewis, B.A., Mangino, M.E, "A Collaborative Study to Develop a Standardized Food Protein Solubility Procedure. Journal of Food Science", Band 50 (1985) Seiten 1715-1718).
   Beim PN-Bestimmungsverfahren wird das Proteinpräparat zu einem Masse-Volumenanteil von 1:25 bis 1:50 (w/v) (d. h. 1-2 g des Proteinpräparats auf 50 ml Lösung) in einer 0,1 M NaCl-Lösung bei Raumtemperatur suspendiert und unter Verwendung von 0,1 M HCl- oder NaOH-Lösung ca. 60 min bei einem pH-Wert von pH 7 gehalten und mit ca. 200 U/min gerührt und das unlösliche Sediment danach für 15 min bei 20tausendfacher Erdbeschleunigung (20'000g) abzentrifugiert. Die Proteinlöslichkeit ist z. B. in Prozent angebbar, wobei eine Proteinlöslichkeit von x % bedeutet, dass x % des im Präparat vorhandenen Proteins im geklärten Überstand wiedergefunden werden, wenn das PN-Bestimmungsverfahren angewendet wird.
- Wasserbindung:
   Das Wasserbindevermögen ist mittels Bestimmungsverfahren (nachfolgend **AACC-Bestimmungsverfahren** genannt) definiert, wie es angegeben ist in: American Association of Cereal Chemists, "Approved methods of the AACC". 10th ed., AACC. St. Paul, MN, 2000b; Method 56-20. "Hydration capacity of pregelatinized cereal products".
   Das Wasserbindevermögen ist z. B. in ml/g TS angebbar, d. h. Milliliter gebundenes Wasser pro Gramm Trockenmasse, und wird gemäss dem AACC-Bestimmungsverfahren bestimmt über das Gewicht des mit Wasser gesättigten Sediments abzüglich der Einwaage des trockenen Präparats nach Mischung von ca. 2 g Proteinpräparat mit ca. 40 ml Wasser für 10 Minuten und Zentrifugation bei 1'000g für 15 Minuten bei 20°C.
- Ölbindung:
   Das Ölbindevermögen ist mittels Bestimmungsverfahren (nachfolgend **OB-Bestimmungsverfahren** genannt) definiert, wie es angegeben ist in: Ludwig I., Ludwig, E., Pingel B., "Eine Mikromethode zur Bestimmung der Fettbindkapazität". Nahrung/Food, 1989, 33(1), 99.
   Das Ölbindevermögen ist z. B. in ml/g angebbar, d. h. Milliliter gebundenes Öl pro Gramm Präparat, und wird gemäss dem OB-Bestimmungsverfahren gemessen als Volumen des ölbindenden Sediments nach Mischung von 1,5 g Proteinpräparat mit 15 ml Maiskeimöl für 1 Minute und Zentrifugation bei 700g für 15 Minuten bei 20°C.
- Emulgierkapazität:
   Die Emulgierkapazität ist mittels Bestimmungsverfahren (nachfolgend **EK-Bestimmungsverfahren** genannt) bestimmt, bei welchem einer 1 %igen Suspension des Proteinpräparats von 100 ml, pH 7, Maiskeimöl zugegeben wird bis zur Phaseninversion der Öl-in-Wasser-Emulsion. Die Emulgierkapazität ist definiert als das maximale Ölaufnahmevermögen dieser Suspension, bestimmt über die spontane Abnahme der Leitfähigkeit bei der Phaseninversion (vgl. den Zeitschriftenartikel von Wäsche, A., Müller, K., Knauf, U., "New processing of lupin protein isolates and functional properties". Nahrung/Food, 2001, 45, 393-395) und ist z. B. angebbar in ml/g, d. h. Milliliter emulgiertes Öl pro Gramm Präparat.
- Schaumaktivität:
   Die Schaumaktivität ist angegeben in Prozent, gemessen als Volumenzunahme einer 5 %igen Lösung, pH 7, bei Aufschlag während 8 min auf Stufe 3 (591 U/min) in einer Hobart 50N Standard-Küchenmaschine (Stahlkessel mit 5 Liter Inhalt) mit Rührbesen (Drahtbesen).
- Schaumdichte:
   Die Schaumdichte ist angegeben in g/l, d. h. Masse des Schaums pro Volumeneinheit, und wird gemessen nach Aufschlag einer 5 %igen Lösung, pH 7, von 8 min auf Stufe 3 (591 U/min) in einer Hobart 50N Standard-Küchenmaschine (Stahlkessel mit 5 Liter Inhalt) mit Rührbesen (Drahtbesen) .
- Schaumstabilität:
   Die Schaumstabilität ist angegeben in Prozent, gemessen als Volumenabnahme von 100 ml Schaum innerhalb einer Stunde nach Aufschlag einer 5 %igen Lösung, pH 7, 8 min auf Stufe 3 (591 U/min) in einer Hobart 50N Standard-Küchenmaschine (Stahlkessel mit 5 Liter Inhalt) mit Rührbesen (Drahtbesen).
- Fettgehalt:
   Der Fettgehalt ist bestimmt nach Probenaufschluss und Verseifung der Fettsäuren z. B. nach der **Caviezel-Methode** (beschrieben bei DGF. "Method of Caviezel", DGF K-I 2c (00). In: DGF-Einheitsmethoden, Deutsche Gesellschaft für Fettwissenschaft e.V., Münster, WVG, Stuttgart, 2nd edition, 2004.).
- Denaturierung:
   Die Denaturierung ist angegeben in Prozent und wird z. B. gemessen durch Vergleich der Denaturierungseigenschaften der Hauptproteinfraktion der Rapskerne vor und nach der Erhitzung mittels "Differential Scanning Calorimetry", wie dies beschrieben ist im Zeitschritenartikel: Sousa, I.M.N., Mitchell, J.R., Ledward, D. A., Hill, S.E., Beirfio da Costa, M.L., "Differential Scanning Calorimetry of lupin and soy proteins". Zeitschrift für Lebensmitteluntersuchung und -Forschung, Band 201 (1995), 566-569.
- Für Vergleichszwecke wurden folgende kommerziell hergestellte Produkte verwendet:
   - Erbsenproteinisolat Pisane® (hergestellt von Cosucra),
   - Sojaproteinisolat SUPRO® EX33 (hergestellt von DuPont).
   - Natriumkaseinat (sprühgetrocknet), FN5S von Rovita.

Mit dem erfindungsgemässen Herstellungsverfahren sind Proteinpräparate herstellbar, welche typischerweise folgende Eigenschaften aufweisen:
Aussehen:
   - In schüttfähiger Form, z. B. als Flocken, Granulat, Pulver oder in Form anderer Partikel.
   - Die Farbe ist weiss bis cremefarben, hellgrau oder hellgelb bis sattgelb, gegebenenfalls mit einem Anteil dunkler gefärbter Partikel von maximal 5 % w/w, vorzugsweise unter 2 % w/w. Die Helligkeit L* gemäss CIE-L*a*b*-Farbmessung ergibt einen Wert von mindestens 80, L* >= 80. Folgendes sind typische Werte für L*, a* und b*: L* >= 80, -5 < a* < +5, -5 < b* < +30; vorzugsweise L* >= 85, -1 < a* < +5, 0 < b* < +25; besonders bevorzugt L* >= 90, -1 < a* < +3, 0 < b* < +20.
Zusammensetzung:
   - Der Proteingehalt beträgt weniger als 90 % in der Trockenmasse (TS) und/oder weniger als 80 % bezogen auf TS. Typischerweise liegt der Proteingehalt zwischen 40 und 70 % bezogen auf TS und/oder zwischen 45 und 65 % bezogen auf TS.
   - Rohfasergehalt zwischen 3 und 20 % bezogen auf TS, bevorzugt zwischen 5 und 10 % bezogen auf TS.
   - Der Gehalt an Gesamtballaststoffen beträgt typischerweise 20-40 %, der Gehalt an unlöslichen Ballaststoffen/ADF ("Acid detergent fibre") typischerweise 8-20 %.
   - Fettgehalt, ermittelt z B. durch gravimetrische Bestimmung nach Soxhletextraktion, typischerweise unter 6 % bezogen auf TS, bevorzugt unter 1 %.
   - Zuckergehalt unter 10 % bezogen auf TS, bevorzugt unter 5 %, besonders bevorzugt unter 2 %.
   - Gehalt an schädlichen, insbesondere antinutritiven Stoffen:
      - Phytinsäuregehalt unter 10 % bezogen auf TS, bevorzugt unter 5 %, besonders bevorzugt unter 3 %.
      - Glucosinolatgehalt (Gehalt an Senfölverbindungen) unter 4000 mg pro kg Proteinpräparat, bevorzugt unter 2500 mg/kg, besonders bevorzugt unter 1500 mg/kg. Insbesondere ist der Progoitringehalt unter 1000 mg/kg, bevorzugt unter 500 mg/kg, besonders bevorzugt unter 200 mg/kg.
      - Phenolsäuregehalt (bestimmt als Sinapinsäure) unter 5 % bezogen auf TS, bevorzugt unter 2 %, besonders bevorzugt unter 0,5 %.
   - Ligningehalt unter 6 % bezogen auf TS, bevorzugt unter 4 %, besonders bevorzugt unter 3 %.
   - Allgemein ist der Protein- sowie Ligningehalt beim Rapsproteinmehl (RPM) tiefer als beim daraus hergestellten Rapsproteinkonzentrat (RPK), während der Gehalt an Fett, Zucker und Phenolsäure beim RPM höher als beim RPK ist.
   - Die Aminosäurezusammensetzung ist ausgewogen mit einer hohen Proteinwertigkeit im Vergleich zu handelsüblichen Pflanzenproteinkonzentraten, wobei der Lysingehalt in Bezug auf das Gesamtprotein mindestens 4 %, bevorzugt mindestens 5 % und der Gehalt schwefelhaltiger Aminosäuren (Summe von Methionin und Cystein) in Bezug auf das Gesamtprotein mindestens 3 %, bevorzugt mindestens 4 % beträgt. Die ursprüngliche Aminosäurezusammensetzung des Rapssamens ist im Wesentlichen beim Proteinpräparat erhalten.
   - Die Denaturierung liegt typischerweise im Bereich von 5 % bis 40 % und/oder im Bereich von 10 % bis 20 %.
Technofunktionelle Eigenschaften:
   - Proteinlöslichkeit:
      Die Proteinlöslichkeit, bestimmt nach dem PN-Bestimmungsverfahren, ist beim RPM grösser als 60 %.
      Typischerweise liegt die Proteinlöslichkeit beim RPM im Bereich bis 70 %, beim RPK im Bereich von 45-55 %.
   - Wasserbindung:
      Das Wasserbindevermögen, bestimmt nach dem AACC-Bestimmungsverfahren, beträgt mindestens 1 ml/g TS, bevorzugt mindestens 2 ml/g TS und besonders bevorzugt mindestens 3 ml/g TS. Vergleichsmessungen zeigen, dass das Wasserbindevermögen des Präparats typischerweise mindestens 30 % des Wasserbindevermögens von Pisane®, bestimmt nach dem AACC-Bestimmungsverfahren, beträgt.
   - Ölbindung:
      Das Ölbindevermögen, bestimmt nach dem OB-Bestimmungsverfahren, beträgt mindestens 1 ml/g, bevorzugt mindestens 2 ml/g und besonders mindestens 4 ml/g.
      Vergleichsmessungen zeigen, dass das Ölbindevermögen mindestens 100% des ölbindevermögens von Pisane® oder von Supro® EX33, bestimmt nach dem OB-Bestimmungsverfahren, beträgt.
   - Emulgierkapazität:
      Die Emulgierkapazität, bestimmt nach dem EK-Bestimmungsverfahren, beträgt mindestens 600 ml/g. Vergleichsmessungen zeigen, dass die Emulgierkapazität mindestens 40% der Emulgierkapazität von Natriumkaseinat FN5S, bestimmt nach dem EK-Bestimmungsverfahren, beträgt. Die Emulgierkapazität ist typischerweise beim RPM höher als beim daraus hergestellten RPK.
   - Schaumbildende Eigenschaften:
      - Schaumaktivität:
         Die Schaumaktivität beträgt mindestens 500 %, vorzugsweise mindestens 1000 %. Vergleichsmessungen mit frischem Hühnereiklar bei Aufschlag während 3 min auf Stufe 3 in einer Hobart 50N Standard-Küchenmaschine mit Rührbesen zeigen, dass die Schaumaktivität des Proteinpräparats mindestens 30 % oder sogar mindestens 60 % der Schaumaktivität von Hühnereiklar entspricht.
      - Schaumdichte:
         Die Schaumdichte liegt im Bereich von 80 und 110 g/l. Vergleichsmessungen mit Hühnereischnee nach Aufschlag während 3 min auf Stufe 3 in einer Hobart 50N Standard-Küchenmaschine mit Rührbesen zeigen, dass die Schaumdichte im Bereich von 80 und 110 % der Schaumdichte von Hühnereischnee liegt.
      - Schaumstabilität:
         Die Schaumstabilität beträgt mindestens 80 %, vorzugsweise mindestens 90 %. Sie entspricht typischerweise mindestens 90 % der Schaumstabilität von Hühnereischnee, gemessen als Volumenabnahme von 100 ml Hühnereischnee innerhalb einer Stunde nach Aufschlag während 3 min auf Stufe 3 in einer Hobart 50N Standard-Küchenmaschine mit Rührbesen.
Sensorische Eigenschaften:
   Nebst der hellen Farbe ist das Proteinpräparat, insbesondere in Form des RPK, im Wesentlichen geruchsfrei und geschmacksneutral. Insbesondere fehlen im Wesentlichen die pflanzen- oder saateigenen Aromen des Rapses. So sind im Wesentlichen kein senfartiger, meerrettichartiger oder stechender Geruch und Geschmack sowie im Wesentlichen kein Bittergeschmack wahrnehmbar.

Sensorische Tests, in welchen geschulte Prüfer einen bestimmten Geschmacks- oder Aromaeindruck des Proteinpräparats und einer geeigneten Referenzsubstanz vergleichen und auf einer Skala von 1 bis 10 (1 = nicht wahrnehmbar, 10 = stark wahrnehmbar) bewerten, wobei die Referenzsubstanz so gewählt ist, dass bei ihr der zu prüfende Geschmacks- oder Aromaeindruck mit mindestens 8 bewertet wird, zeigen, dass dem Proteinpräparat ein Wert von 3 oder weniger (typischerweise ein Wert von 1) zugeordnet wird.

Beispiele von zu testenden Geschmacks- oder Aromaeindrücken sind:
- meerrettichartiger/stechender Geruch und Geschmack im Vergleich zu einem marktüblichen handelsüblichen Markenmeerrettich, z. B. "Meerrettich" von Hengstenberg,
- senfartiger Geruch und Geschmack im Vergleich zu einem handelsüblichen Markensenf, z. B. "Löwensenf medium",
- Bittergeschmack im Vergleich zu einer 0,1 %igen wässrigen Koffeinlösung,
- Adstringenz (raues, pelziges Mundgefühl) im Vergleich zu einem herben Rotwein oder einer Bitterschokolade.

Farbe, Eigengeschmack und Eigengeruch des Proteinpräparats in Form des RPK sind so, dass bei der Einarbeitung in Lebens- und Futtermittel im Wesentlichen keine mit üblichen statistischen Methoden ermittelte signifikante, als negativ zu wertende Veränderung des arteigenen Aussehens, Geruchs und Geschmacks der fertigen Zubereitung auftritt.

Sensorische Tests zeigen, dass die Geschmacks- und Aromaänderung, die in einer Lebensmittelzubereitung durch den Einsatz des Proteinpräparats hervorgerufen wird, gegenüber der Lebensmittelzubereitung ohne das Proteinpräparat auf ein derartiges Mass begrenzt ist, dass ein geschulter Prüfer eine Abweichung eines der oben genannten Geschmacks- oder Aromamerkmale (Meerrettich, Senf, etc.) auf einer Skala von 1-10 von maximal 3 Stufen oder sogar maximal 1 Stufe (fast nicht mehr erkennbare Abweichung) erkennen kann.

### C) Beispiele

Nachfolgend werden Herstellungsverfahren und Proteinpräparat gemäss der Erfindung an weiteren Beispielen illustriert.

### C.1) Beispiel 1: Rapsproteinmehl und Rapsproteinkonzentrat aus 90 % geschälten Rapssamen

### (Beispiel nicht Teil der Erfindung)

Herstellung:
1. Schälung der Rapssamen durch Aufschluss in einer Prallmühle und Auftrennung in eine kernreiche und schalenreiche Fraktion im Luftstrom.
2. Siebung der Grobfraktionen mit einem Sieb der Maschenweite 1,25 mm, um ungeschälte Kerne zu entfernen und im Siebdurchgang eine aufgereinigte Kernfraktion zu erhalten. (Die Kernfraktion enthält bei diesem Beispiel einen Schalenanteil von ca. 10 % w/w).
3. Flockierung der aufgereinigten Kernfraktion mittels Glattwalzen, um Rapskernflakes zu erhalten.
4. Entölung der Rapskernflakes im Soxhlet bei Temperaturen von maximal 80°C, überwiegend unter 60°C.
5. Entfernung des Lösemittels im Luftstrom bei Raumtemperatur. Das so erhaltene Raffinat ergibt das nachfolgend erwähnte Rapsproteinmehl.
6. Dreifache Extraktion des Rapsproteinmehls mit dem 10 fachen Volumen z. B. einer 80 %igen (v/v) IsoPropanol-Lösung bei Raumtemperatur für jeweils 1 h unter Rühren und Abtrennung des Extrakts über ein Filtertuch.
7. Nach der dritten Extraktion werden die Lösemittelrückstände durch Lufttrocknung entfernt.
8. Vermahlung des im obigen Verfahrensschritt 7 erhaltenen Raffinats in einer Stiftmühle mit Siebeinsatz von 1 mm.
9. Nachtrocknung des Raffinats im Vakuum bei maximal 50°C.
10. Sieben des Raffinats mit einem Sieb der Maschenweite von 315 Mikrometer, um ein Rapsproteinkonzentrat als feines Pulver zu erhalten.

Eigenschaften:
Das so erhaltene Rapsproteinkonzentrat hat einen Proteingehalt von mehr als 55 % in TS. Die Zusammensetzung ist in unten stehender Tabelle 1, einige funktionelle Eigenschaften sind in den unten stehenden Tabelle 2 und 3 aufgelistet. In diesen drei Tabellen sind auch die Werte für das beim oben erwähnten Verfahrensschritt 5 erhaltende Proteinpräparat in Form des Rapsproteinmehls angegeben.

Durch Variation der Alkoholart (Methanol, Ethanol, IsoPropanol) und des Alkoholgehalts (z.B. 70, 80, 90 % v/v) beim oben erwähnten Verfahrensschritt 6 wurden nach demselben Verfahren ähnliche Proteinpräparate hergestellt. Die Farbe dieser unterschiedlichen Rapsproteinkonzentrate wurde nach CIE L*a*b* bestimmt, wobei sich folgende Mittelwerte und Standardabweichungen ergaben:

| | L* | a* | b* |
|---|---|---|---|
| Mittelwert aller Proteinkonzentrate | 73,5 | 2,6 | 22,0 |
| Standardabweichung | 4,7 | 1,3 | 2,9 |

### C.2) Beispiel 2: Rapsproteinmehl und Rapsproteinkonzentrat aus 100% geschälten Rapssamen

Herstellung:
1. Schälung der Rapssamen durch Aufschluss in einer Prallmühle und Auftrennung in eine kernreiche Grobfraktion und schalenreiche Feinfraktion im Luftstrom.
2. Siebung der Grobfraktionen mit einem Sieb der Maschenweite 1,25 mm, um ungeschälte Kerne zu entfernen und im Siebdurchgang eine vorgereinigte Kernfraktion zu erhalten.
3. Aussortieren von Schalenpartikeln und ungeschälten Kernen aus der vorgereinigten Kernfraktion durch Farbsortierung, um eine reine Kernfraktion zu erhalten. Die Kernfraktion ist demnach nahezu frei von Schalen.
4. Flockierung der Kernfraktion im Walzenstuhl mit zwei gegenläufigen Glattwalzen, die dazwischen einen Abstand von 0,5 mm aufwiesen.
5. Entölung der Rapskernflakes mit n-Hexan im Soxhlet bei Temperaturen von maximal 80°C, überwiegend unter 60°C.
6. Entfernung des Lösemittels im Luftstrom bei Raumtemperatur. Das so erhaltene Raffinat ergibt das nachfolgend erwähnte Rapsproteinmehl.
7. Dreifache Extraktion des Rapsproteinmehls mit dem 10 fachen Volumen einer 70 %igen (v/v) Ethanol-Lösung bei Raumtemperatur für jeweils 1 h unter Rühren und Abtrennung des Extrakts über ein Filtertuch.
8. Zweifache Extraktion des aus dem Verfahrensschritt 7 erhaltenen Raffinats mit 100 % Ethanol, um die Alkoholkonzentration im Raffinat auf mindestens 90% zu erhöhen.
9. Verdampfung des Alkohols und Trocknung des im Verfahrensschritt 8 erhaltenen Raffinats im Rotationsverdampfer unter Vakuum bei maximal 50°C, um ein Rapsproteinkonzentrat zu erhalten.
10. Vermahlung des Rapsproteinkonzentrats in einer Stiftmühle mit Siebeinsatz 0,5 mm, um das Rapsproteinkonzentrat als feines Pulver zu erhalten.

Eigenschaften:
Das so erhaltene Rapsproteinkonzentrat ist eine feines helles Pulver mit einem Proteingehalt von mehr als 55 % in TS. Die Zusammensetzung ist in Tabelle 1, einige funktionelle Eigenschaften sind in der Tabelle 2 aufgelistet. In diesen zwei Tabellen sind auch die Werte für das beim oben erwähnten Verfahrensschritt 6 erhaltende Rapsproteinmehl angegeben.

Das so erhaltene Rapsproteinkonzentrat ist frei von rapseigenen oder senf- und meerrettichartigen Aromakomponenten.

Die Farbe des im Verfahrensschritt 6 erhaltenen, schalenfreien Rapsproteinmehls und des im Verfahrensschritt 10 erhaltenen Rapsproteinkonzentrats ist besonders ansprechend, d. h. neutral und weist nach CIE-L*a*b* folgende Werte auf:

| | L* | a* | b* |
|---|---|---|---|
| Rapsproteinmehl | 87,8 | +0,3 | +25,7 |
| Rapsproteinkonzentrat | 90,3 | -0,2 | +16,3 |

### C.3) Beispiel 3: Rapsproteinmehl und Rapsproteinkonzentrat aus geschälten Rapssamen

Herstellung:
1. Schälung der Rapssamen durch Aufschluss in einer Prallmühle und Auftrennung in eine kernreiche Grobfraktion und eine schalenreiche Feinfraktion im Luftstrom in einem Zickzacksichter (Sichter mit besonders guter Trennschärfe, indem der Kanal, in welchem der Luftstrom aufsteigt, durch im Zickzack angeordnete Wände in mehrere kleinere Röhren unterteilt ist) .
2. Siebung der Grobfraktionen mit einem Taumelsieb der Maschenweite 1,5 mm, um ungeschälte Kerne zu entfernen und im Siebdurchgang eine gereinigte Kernfraktion zu erhalten
3. Flockierung der Kernfraktion zu "Rapskernflakes" im Walzenstuhl mit zwei gegenläufigen Glattwalzen, die dazwischen einen Abstand von 0,3 mm aufwiesen, bei Temperaturen unter 30°C.
4. Entölung der Rapskernflakes mit iso-Hexan in einem Perkolator bei Temperaturen von maximal 60°C.
5. Austreiben des Hexans mit überhitztem Hexandampf unter Vakuum (< 500 mbar).
6. Austreiben weiteren Hexans mit überhitztem Wasserdampf unter Vakuum (< 500 mbar).
7. Entfernung von Lösemittelresten durch Erhitzung auf 60°C im Vakuum (< 500 mbar). Das so erhaltene Raffinat wird nachfolgend Proteinmehl genannt.
8. Dreifache Extraktion des Proteinmehls mit der 5-8fachen Masse einer 65 %igen (w/w) Ethanol-Lösung bei Raumtemperatur unter Umwälzung des Lösemittels bis zur Dichtekonstanz und Abtrennung des Extrakts über einen Siebboden.
9. Zweifache Extraktion des Raffinats mit 94 % w/w Ethanol, um die Alkoholkonzentration im Raffinat auf mindestens 90 % w/w zu erhöhen.
10. Verdampfung des Alkohols und Trocknung des so erhaltenen Raffinats durch Erhitzung auf 50-60°C unter Vakuum (< 300 mbar), um ein Proteinkonzentrat zu erhalten.
11. Vermahlung des Proteinkonzentrats in einer Stiftmühle mit Siebeinsatz 0,5 mm, um das Proteinkonzentrat als feines Pulver zu erhalten.

Eigenschaften:
Das so erhaltene Rapsproteinkonzentrat ist ein feines helles Pulver mit einem Proteingehalt von > 60 % in TS. Die Zusammensetzung ist in Tabelle 1 des Abschnittes D, einige funktionelle Eigenschaften sind in den Tabellen 2 und 3 aufgelistet.

Das so erhaltene Proteinkonzentrat ist arm an rapseigenen oder senf- und meerrettichartigen Aromakomponenten.

Die Farbe des so hergestellten schalenarmen Rapsproteinmehls (RPM) und Rapsproteinkonzentrats (RPK) ist besonders ansprechend bzw. neutral und wird nach CIE L*a*b* mit folgenden Werten wiedergegeben:

| | L* | a* | b* |
|---|---|---|---|
| Rapsproteinmehl | 84,8 | -1,7 | +26,7 |
| Rapsproteinkonzentrat | 86,2 | -0,1 | +17,4 |

### C.4) Beispiel 4: Rapsproteinmehl und Rapsproteinkonzentrat mit spezifischen Eigenschaften

Bei diesem Beispiel wurde unter anderem die Modifizierung der funktionellen Eigenschaften des Rapsproteinpräparats untersucht bei einer abschliessenden Aufbereitung in der Korngrösse.

Herstellung:
1. Schälung der Rapssamen durch Aufschluss in einer Prallmühle und Auftrennung in eine kernreiche Grobfraktion und eine schalenreiche Feinfraktion im Luftstrom in einem Zickzacksichter.
2. Siebung der Grobfraktionen mit einem Taumelsieb der Maschenweite 1,5 mm, um ungeschälte Rapssamen zu entfernen und im Siebdurchgang eine gereinigte Kernfraktion zu erhalten.
3. Flockierung der Kernfraktion im Walzenstuhl mit zwei gegenläufigen Glattwalzen, die dazwischen einen Abstand von 0,3 mm aufwiesen, bei Temperaturen unter 30°C.
4. Entölung der Rapskernflakes mit iso-Hexan in einem Perkolator bei Temperaturen von maximal 60°C.
5. Austreiben des Hexans mit überhitztem Hexandampf unter Vakuum (< 500 mbar).
6. Austreiben weiteren Hexans mit überhitztem Wasserdampf unter Vakuum (< 500 mbar).
7. Entfernung von Lösemittelresten durch Erhitzung auf 60°C im Vakuum (< 500 mbar). Das so erhaltene Raffinat wird nachfolgend Proteinmehl genannt.
8. Sichtung, Siebung und/oder Vermahlung des Proteinmehls in einer Stift- oder Schlagmühle, um Fraktionen mit unterschiedlichem Schalengehalt und/oder unterschiedlicher Partikelgrössenverteilung zu erhalten und so die funktionellen Eigenschaften zu modifizieren. Die Einstellung der Mühlen sind in der Tabelle 4 im Abschnitt D angegeben.
9. Dreifache Extraktion des Proteinmehls mit der 5-8fachen Masse einer 65 %igen (w/w) Ethanol-Lösung bei Raumtemperatur unter Umwälzung des Lösemittels bis zur Dichtekonstanz und Abtrennung des Extrakts über einen Siebboden.
10. Zweifache Extraktion des Raffinats mit 94 % w/w Ethanol, um die Alkoholkonzentration im Raffinat auf mindestens 90 % zu erhöhen
11. Verdampfung des Alkohols und Trocknung des so erhaltenen Raffinats durch Erhitzung auf 50-60°C unter Vakuum (< 300 mbar), um ein Proteinkonzentrat zu erhalten.
12. Sichtung, Siebung und/oder Vermahlung des Proteinkonzentrats in einer Stift- oder Schlagmühle (Einstellungen siehe Tabelle 4), um Fraktionen mit unterschiedlichem Schalengehalt und/oder unterschiedlicher Partikelgrössenverteilung zu erhalten und so die funktionellen Eigenschaften zu modifizieren

Durch eine Aufbereitung in der Korngrösse, wie sie oben im Schritt 8 oder Schritt 12 durchgeführt wurde, waren die funktionellen Eigenschaften des Proteinpräparates veränderbar. Zur Korngrössenverkleinerung wurde neben einer reinen Vermahlung auch eine Sichtung oder eine Siebung, gegebenenfalls in Verbindung mit einer Vermahlung eingesetzt. Wie aus der Tabelle 4 ersichtlich, nimmt mit abnehmender Korngrösse die Wasserbindung tendenziell zu, ebenso die Emulgierkapazität beim Rapsproteinkonzentrat, während die Ölbindung geringfügig abnahm oder sich kaum änderte. Wie aus Tabelle 4 weiter ersichtlich, haben die Präparate mit einer homogeneren Korngrössenverteilung eine höhere Wasserbindung. Als besonders vorteilhaft zur Steigerung der Wasserbindung stellte sich die Kombination von Fraktionierung und Zerkleinerung heraus. Insgesamt ist es möglich, das funktionelle Profil durch eine zielgerichtete Aufbereitung in der Korngrössenverteilung zu modifizieren.

Bei den Beispielen 1 bis 4 wurde als mechanische Vorbehandlung eine Flockierung gewählt, mit welcher die Kerne in eine für die nachfolgende Behandlung günstige Form gebracht wurden. Die Kernfraktion wurde anschliessend alleine durch Verwendung eines Lösemittels entölt. Eine vorgängige mechanische Entölung wurde nicht durchgeführt. Es ist jedoch vorteilfhaft, diese vorzusehen, wenn Proteinpräparate besonders kostengünstig und/oder in grösserem Umfang herzustellen sind. Weitere Vorteile einer mechanischen Entölung ergeben sich aus dem nachfolgend beschriebenen Beispiel 5.

### C.5) Beispiel 5: Rapsproteinmehl und Rapsproteinkonzentrat aus geschälten Rapssamen

Herstellung:
1. Schälung der Rapssamen durch Aufschluss in einer Schlagmühle und Auftrennung in eine kernreiche Grobfraktion mit einem Schalenanteil von unter 3 % w/w und eine schalenreiche Feinfraktion im Luftstrom in einem Zickzacksichter.
2. Pressung der Kernfraktion in einer Schneckenpresse auf einen Restfettgehalt von ca. 23 % w/w bei Temperaturen zwischen 30 und 45°C, wobei der Presskuchen in Form von zusammengepressten Strängen, nachfolgend Presskuchen-Pellets genannt, erhalten wird.
3. Entölung der Presskuchen-Pellets mit Hexan in einer Soxhlet-Apparatur auf einen Restfettgehalt unter 3 % w/w.
4. Entfernung des Lösemittels im Luftstrom bei Raumtemperatur. Das so erhaltene Raffinat liegt weiterhin in Form der Pellets vor und entspricht in seiner Zusammensetzung dem Proteinmehl wie in Beispiel 1.
5. Extraktion der Proteinmehl-Pellets aus dem Schritt 4 ohne weitere Zerkleinerung, indem sie mit einer Ethanol-Lösung im Perkolationsverfahren mit Rückführung des Lösemittels behandelt werden, bis das Lösemittel keine Änderung mehr aufweist.
6. Wiederholung von Schritt 5 mit frischem Lösemittel.
7. weitere Behandlung wie in Beispiel 1, Schritt 7-9.
8. Einsatz des fertigen Proteinkonzentrats mit oder ohne nachfolgende Zerkleinerung.

Bei diesem Beispiel 5 lagen die Rapskerne nach der Schälung schon in einer solchen Form vor, dass sie direkt mechanisch entölt werden konnten. Eine vorherige Zerkleinerung oder Flockierung wurde nicht durchgeführt.

Die Entölung durch Pressen wurde beim Schritt 2 mit einer Schneckenpresse durchgeführt. Bei einer Austrittstemperatur von ca. 40°C wurden am Düsenausgang der Schneckenpresse runde Presskuchen-Pellets mit einem Fettgehalt von 23 % w/w (25 % in TS) erhalten, die eine hohe Porosität und einen guten Zusammenhalt und eine mechanische Stabilität aufwiesen, so dass sie direkt zur Entölung eingesetzt werden konnten und bei der Entölung gerade noch nicht auseinanderfielen. Bei einem höheren Pressgrad auf einen Restfettgehalt von ca. 17 % w/w waren diese Pellets etwas dichter und fester und hatten eine höhere mechanische Stabilität, konnten mit mässigem Kraftaufwand aber noch auseinandergebrochen werden. Bei einem noch höheren Pressgrad auf niedrigere Fettgehalte waren die Pellets sehr fest und stabil und konnten nur z. B. mithilfe einer Mühle zerbrochen werden.

Im Weiteren wurde festgestellt, dass bei einem zu hohen Pressgrad die Pellets eine dunkle Verfärbung aufwiesen, was auf unerwünschte Proteinschäden schliessen lässt und die Farbe des Proteinpräparats beeinträchtigen würde.

Es wurde erkannt, dass bei einem Pressgrad auf einen Restfettgehalt im Bereich von typischerweise ca. 17 % bis 25 % w/w Restfett Presskuchen-Pellets erhalten werden, die eine gute mechanische Stabilität bei noch ausreichend vorhandener Porosität aufweisen, so dass ohne weitere Strukturierung oder Zerkleinerung bei der nachfolgenden Extraktion eine vollständige Entölung möglich ist.

Überraschenderweise weisen die Pellets auch nach der Entölung trotz der mit der Entfernung des Öls einhergehenden Lockerung der Struktur noch eine ausreichende mechanische Stabilität auf, so dass sie einer Extraktion mit einem weiteren Lösemittel unterworfen und so an proteinfremden Substanzen abgereichert werden können, ohne auseinanderzufallen. Durch die poröse Struktur haben sie ein sehr günstiges Extraktionsverhalten für die weitere Extraktion mit alkoholischer Lösung.

Insgesamt können durch ein Pressen, bei welchem ein bestimmter Restfettgehalt bestehen bleibt, die Partikel so strukturiert werden, dass eine nachträgliche Strukturierung oder Zerkleinerung nicht mehr erforderlich ist, die sonst üblicherweise zum Aufbrechen des Presskuchens durchgeführt wird. Neben der Vereinfachung des Verfahrens trägt dies zur Schonung des Presskuchens bei, so dass u. a. die Proteinfunktionalität und die Farbe im Endprodukt verbesserbar sind.

Auch werden durch den reduzierten Pressgrad die Proteine geschont und die funktionellen Eigenschaften des Proteinpräparats bleiben in verbessertem Masse erhalten. Dabei wird gleichzeitig eine Partikelform hergestellt, die eine optimale Extraktion ermöglicht und so der Restölgehalt nach dem Entölen weiter gesenkt werden kann. Auch dies trägt u. a. zu einer Verbesserung der Farbe des Proteinpräparats bei.

### D) Tabellen

**Tabelle 1: Zusammensetzung der Rapsproteinpräparate aus den Beispielen 1 bis 3 im Vergleich zu Referenzprodukten. Die angegebenen Werte für den Gehalt an Proteinen, Asche, Fett und Glucose beziehen sich auf die Trockenmasse (TS). Der Proteingehalt wurde durch Bestimmung des Stickstoff mittels Dumas-Methode und Multiplikation mit dem Faktor 6,25 ermittelt. Die Asche wurde mit TGA-Bestimmung 950°C ermittelt. Die Angabe des Fettgehaltes ist inkl. Phospholipide nach Caviezel. Der Glucosegehalte wurde photometrisch nach einfacher Extraktion bestimmt.**

| | TS | Protein (N×6,25) | Asche | Gesamtfett | Glucose |
|---|---|---|---|---|---|
| | % | % (TS) | % (TS) | % (TS) | % (TS) |
| Rapsproteinmehl aus Beispiel 1 | 91,0 | 49,0 | 8,4 | 2,8 | |
| Proteinkonzentrat extrahiert mit 80% Isopropanol (Beispiel 1) | 91,6 | 59,5 | 9,5 | 0,7 | |
| Rapsproteinmehl aus Beispiel 2 | 90,6 | 46,8 | 8,4 | 1,7 | 7,8 |
| Proteinkonzentrat extrahiert mit 70% EtOH (Beispiel 2) | 83,1 | 59,3 | 8,8 | 0,5 | 1,4 |
| Rapsproteinmehl aus Beispiel 3 | 89,5 | 51,4 | 8,2 | 5,8 | |
| Proteinkonzentrat aus Beispiel 3 | 89,8 | 61,8 | 9,7 | 2,4 | |
| Sojaproteinisolat Supro® Ex 33 | 94,7 | 92,2 | 3,0 | | |
| Pisane® Erbsenisolat | 94,9 | 89,0 | 5,1 | | |

**Tabelle 2:**

| Funktionelle Eigenschaften der Rapsproteinpräparate aus den Beispielen 1 bis 3 im Vergleich zu Referenzprodukten. Die Präparate wurden zur Analyse der funktionellen Eigenschaften in einer Labor-Schlagmühle mit Siebeinsatz 500 *µ*m vermahlen. | | | | |
|---|---|---|---|---|
| | Proteinlöslichkeit pH 7 | Emulgierkapazität | Wasserbindevermögen | Ölbindevermögen |
| | % | ml/g | ml/g (TS) | ml/g |
| Rapsproteinmehl aus Beispiel 1 | 72 | 660 | | |
| Proteinkonzentrat extrahiert mit 80% Isopropanol (Beispiel 1) | 54 | 510 | | |
| Rapsproteinmehl aus Beispiel 2 | 75 | 655 | 3,2 | 2,7 |
| Proteinkonzentrat extrahiert mit 70% EtOH (Beispiel 2) | 57 | 475 | 2,4 | 4,7 |
| Rapsproteinmehl aus Beispiel 3 | 73 | 675 | 3,4 | 2,7 |
| Proteinkonzentrat aus Beispiel 3 | 47 | 450 | 4,8 | 2,3 |
| Sojaproteinisolat Supro® Ex 33 | | 635 | | 1,5 |
| Pisane® Erbsenisolat | | 350 | 6,3 | 1,9 |

**Tabelle 3:**

| Schaumbildende Funktionalitäten der Rapsproteinpräparate aus den Beispielen 1 und 3 im Vergleich zu frischem Hühnereiklar. Die Präparate wurden zur Analyse der schaumbildenden Eigenschaften in einer Labor-Schlagmühle mit Siebeinsatz 500 *µ*m vermahlen. | | | |
|---|---|---|---|
| | Schaum- aktivität | Schaum- stabilität | Schaumdichte |
| | % | % | g/l |
| Rapsproteinmehl aus Beispiel 1 | 1320 | 94 | 81 |
| Proteinkonzentrat extrahiert mit 80% Isopropanol (Beispiel 1) | 1100 | 86 | 88 |
| Rapsproteinmehl aus Beispiel 3 | 540 | 88 | 184 |
| Proteinkonzentrat aus Beispiel 3 | 1070 | 93 | 109 |
| Hühnereiklar, frisch | 1600 | 82 | 105 |

**Tabelle 4:**

| | | | | | | |
|---|---|---|---|---|---|---|
| Funktionelle Eigenschaften von Rapsproteinmehlen (RPM) und Rapsproteinkonzentraten (RPK), welche in der Korngrösse unterschiedlich aufbereitet sind. Die Spalten S1-S2 haben folgende Bedeutung: | | | | | | |
| S1: | Korngrösse des fertigen Proteinpräparats in Mikrometer. | | | | | |
| S2: | Homogenität, wobei "0" eine breite, "+" eine engere, "++" eine noch engere und "+++" eine sehr enge Korngrössenverteilung bedeutet. | | | | | |
| S3: | Wasserbindevermögen in ml/g (TS) | | | | | |
| S4: | Ölbindevermögen in ml/g | | | | | |
| S5: | Emulgierkapazität in ml/g | | | | | |
| Leere Kästchen in den Spalten S1-S2 bedeuten, dass der entsprechende Wert nicht bestimmt worden ist. | | | | | | |

| | Methode | S1 | S2 | S3 | S4 | S5 |
|---|---|---|---|---|---|---|
| RPM | unbehandelt | >1000 | 0 | 2,5 | 3,1 | 680 |
| | gemahlen < 500 *µ*m | <500 | + | 3,4 | 2,7 | 675 |
| RPK | gemahlen < 500 *µ*m | <500 | + | 4,8 | 2,3 | 450 |
| | 2fach gemahlen < 500 *µ*m | <500 | ++ | 5,0 | 2,1 | |
| | gesiebt: < 560 *µ*m | <560 | + | 4,7 | 2,0 | 465 |
| | gesiebt < 560 *µ*m und anschliessend gemahlen < 500 *µ*m | <500 | +++ | 5,6 | | 475 |
| | Feingut aus Sichtung (0,3-0,4) | | + | 5,3 | 2,1 | 475 |
| | Feingut aus Sichtung (0,3-0,4), gemahlen (500 *µ*m) | <500 | +++ | 5,7 | | |

Aus der vorangehenden Beschreibung sind dem Fachmann zahlreiche Abwandlungen zugänglich, ohne den Schutzbereich der Erfindung zu verlassen, der durch die Ansprüche definiert ist.

So ist es z. B. denkbar, das erfindungsgemässe Proteinpräparat als Ausgangsstoff zur Herstellung eines Proteinisolates zu verwenden, bei welchem der Proteingehalt mindestens 90 % in TS beträgt. Die Herstellung erfolgt z. B. mittels wässriger Extraktion der Proteine.

Weiter ist es denkbar, die bei der Schälung anfallenden Schalenfraktionen so aufzubereiten, dass sie energetisch verwertbar sind, um Strom und/oder nutzbare Wärme zu erzeugen. Die Aufbereitung der Schalenfraktionen kann u. a. so erfolgen, dass die Schalen entölt werden, z. B. durch Pressen und/oder mittels Lösemittel, und/oder pelletiert werden. Die energetische Verwertung kann z. B. durch Verbrennen erfolgen.

Gegebenenfalls werden den Schalen Holz und/oder eine andere Art geeigneter Biomasse beigemischt, um die Verbrennung zu verbessern. Diese Biomasse kann auch von der Aufbereitung der Rapssamen herrühren, bevor diese geschält werden, und z. B. in Form des Besatzes bei der Rohstofflieferung (Bruchstücke, Verunreinigungen, etc.) und/oder in Form des Unterkorns und Überkorns sein (Teil der Rapssamen, welcher vor der Schälung entfernt wird, um für die Schälung Rapssamen mit einer möglichst einheitlichen Grösse zu erhalten.)

Vorzugsweise werden den zu verbrennenden Schalen Rapsbestandteile, die von den beim Schälen der Rapssamen anfallenden Kerne oder aus diesen Kernen gewonnenen Fraktionen stammen, nicht oder nur in beschränktem Masse beigefügt, so dass das Verhältnis der Masse der Rapsbestandteile zur Masse der Schalen kleiner als 1 zu 1, bevorzugt kleiner als 1 zu 2 und besonders bevorzugt kleiner als 1 zu 5 ist.

### E) Glossar:

mg: Milligramm
ml: Milliliter
RPK: Rapsproteinkonzentrat
RPM: Rapsproteinmehl
TS: Trockenmasse
Prozent v/v ("volume per volume"): Volumenprozent, bestimmt bei einer Temperatur von 25 Grad Celsius (Volumenanteil einer Komponente am Volumen eines Gemisches)
Prozent w/w ("weight per weight"):
Masseprozent/Gewichtsprozent

## Patentansprüche

1. Proteinpräparat, welches ein Rapsproteinmehl ist, das aus Rapssamen hergestellt ist, wobei das Proteinpräparat
einen Proteingehalt aufweist, der bezogen auf die Trockenmasse mindestens 40 % und höchstens 70 % beträgt, wobei
das Proteinpräparat eine Helligkeit L*, bestimmt gemäss CIE-L*a*b*-Farbmessung, von mindestens 80 aufweist sowie zumindest wasserbindende, ölbindende und emulgierende Funktionalität, wobei
die Emulgierkapazität, bestimmt nach dem in der Beschreibung definierten EK-Bestimmungsverfahren, mindestens 600 ml/g beträgt und wobei
das Proteinpräparat einen Fettgehalt aufweist, der, bezogen auf die Trockenmasse und bestimmt nach Caviezel, unter 6 % liegt, und
eine Proteinlöslichkeit, die, bestimmt nach dem in der Beschreibung definierten PN-Bestimmungsverfahren, grösser als 60 % ist.

2. Proteinpräparat nach Anspruch 1, wobei die Helligkeit L* mindestens 85 beträgt, bevorzugt mindestens 90, und/oder
die Werte für a* und b* gemäss CIE-L*a*b*-Farbmessung im Bereich von
-5 < a* < +5, -5 < b* < +30 liegen, vorzugsweise im Bereich von -1 < a* < +5, 0 < b* < +25 und besonders bevorzugt im Bereich von -1 < a* < +3, 0 < b* < +20.

3. Proteinpräparat nach einem der vorangehenden Ansprüche, wobei das Wasserbindevermögen des Proteinpräparats, bestimmt nach dem in der Beschreibung definierten AACC-Bestimmungsverfahren, mindestens 1 ml pro Gramm Trockenmasse beträgt, bevorzugt mindestens 2 ml pro Gramm Trockenmasse und besonders bevorzugt mindestens 3 ml pro Gramm Trockenmasse, und/oder
das Ölbindevermögen, bestimmt nach dem in der Beschreibung definierten OB-Bestimmungsverfahren, mindestens 1 ml/g beträgt, bevorzugt mindestens 2 ml/g.

4. Proteinpräparat nach einem der vorangehenden Ansprüche, wobei das Proteinpräparat zusätzlich schaumbildende Funktionalität aufweist.

5. Proteinpräparat nach Anspruch 4, wobei das Proteinpräparat mindestens eine der folgenden schaumbildenden Eigenschaften aufweist:
- die Schaumaktivität entspricht mindestens 30 %, bevorzugt mindestens 50 % der Schaumaktivität von Hühnereiklar,
- die Schaumdichte entspricht mindestens 50 % und/oder höchstens 200 % der Schaumdichte von Hühnereischnee,
- die Schaumstabilität entspricht mindestens 80 %, bevorzugt mindestens 90 % und besonders bevorzugt mindestens 100 % der Schaumstabilität von Hühnereischnee.

6. Proteinpräparat nach einem der vorangehenden Ansprüche, wobei der Proteingehalt, bezogen auf die Trockenmasse, mindestens 45 % beträgt und/oder höchstens 65 %.

7. Proteinpräparat nach einem der vorangehenden Ansprüche, wobei das Proteinpräparat einen Rohfasergehalt aufweist, der, bezogen auf die Trockenmasse, mindestens 3 %, bevorzugt mindestens 5 %, beträgt und/oder höchstens 30 %, bevorzugt höchstens 20 % und besonders bevorzugt höchstens 10 %.

8. Proteinpräparat nach einem der vorangehenden Ansprüche, wobei das Proteinpräparat einen Fettgehalt aufweist, der, bezogen auf die Trockenmasse und bestimmt nach Caviezel, unter 5 % liegt, bevorzugt unter 3 % liegt.

9. Proteinpräparat nach einem der vorangehenden Ansprüche, wobei das Proteinpräparat mindestens einen Gehalt, bevorzugt alle der folgenden Gehalte an Stoffen aufweist:
- Phytinsäuregehalt, bezogen auf die Trockenmasse, von unter 10 %, bevorzugt unter 5 %, besonders bevorzugt unter 3 %,
- Glucosinolatgehalt von unter 4000 mg pro kg Proteinpräparat, bevorzugt unter 2500 mg/kg, besonders bevorzugt unter 1500 mg/kg,
- Progoitringehalt von unter 1000 mg pro kg Proteinpräparat, bevorzugt unter 500 mg/kg, besonders bevorzugt unter 200 mg/kg,
- Phenolsäuregehalt, bezogen auf die Trockenmasse, von unter 5 %, bevorzugt unter 2 %, besonders bevorzugt unter 0,5 %.

10. Verfahren zur Herstellung eines Proteinpräparates aus Rapssamen, welches ein Rapsproteinmehl ist, mit folgenden Schritten:
- Schälen der Rapssamen zum Erhalt einer Kernfraktion mit einem Schalenanteil von weniger als 5 % w/w,
- mechanische Behandlung der Kernfraktion, in welcher die Form der Kerne verändert wird, und
- Lösemittel-Entölung der mechanisch behandelten Kernfraktion mittels Lösemittel zum Erhalt des Rapsproteinmehls,
wobei die mechanische Behandlung eine mechanische Entölung durch Pressen der Kernfraktion umfasst, in welcher ein Teil des Öls abgetrennt wird, so dass die mechanisch entölte Kernfraktion einen Restölgehalt von mindestens 15 % w/w und höchstens 35 % w/w aufweist, und wobei die über die Zeitdauer des Pressvorganges gemittelte Temperatur Tm der Kernfraktion unter 60 Grad Celsius liegt.

11. Verfahren nach Anspruch 10, wobei die zur mechanischen Behandlung verwendete Kernfraktion einen Schalenanteil aufweist von weniger als 1 % w/w.

12. Verfahren nach einem der Ansprüche 10 bis 11, wobei die mechanische Behandlung weiter eine Flockierung der Kerne umfasst.

13. Verfahren nach einem der Ansprüche 10 bis 12, wobei die Lösemittel-Entölung bei einer ersten Temperatur durchgeführt wird und anschliessend das Lösemittel bei einer zweiten Temperatur entfernt wird, wobei die erste Temperatur unter 90 Grad Celsius liegt, bevorzugt unter 60 Grad Celsius, und/oder wobei die zweite Temperatur unter 90 Grad Celsius liegt, bevorzugt unter 70 Grad Celsius.

14. Verfahren nach einem der Ansprüche 10 bis 13, wobei die mechanisch entölte Kernfraktion einen Restölgehalt aufweist von höchstens 25 % w/w, bevorzugt höchstens 20 % w/w.

15. Verfahren nach einem der Ansprüche 10 bis 14, wobei zum Erhalt eines Proteinkonzentrates aus dem Rapsproteinmehl eine Extraktion durchgeführt wird, in welcher die proteinfremden Stoffe mittels eines Extraktionslösungsmittels aus dem Proteinmehl abgereichert werden und die Proteine weitgehend ungelöst bleiben, wobei das nach der Extraktion erhaltene Proteinkonzentrat in der Korngrösse aufbereitet wird, um ein Schüttgut mit einer vorgegebenen Korngrössenverteilung zu erhalten.

16. Verfahren nach Anspruch 15, wobei bei der Extraktion ein wässrig-alkoholisches Extraktionslösungsmittel verwendet und die Extraktion in mehreren Extraktionsschritten durchgeführt wird, wobei bei mindestens einem Übergang von dem einem zum nächsten Extraktionsschritt der Alkoholgehalt im Extraktionslösungsmittel erhöht wird.

17. Verfahren nach Anspruch 15 oder 16, wobei die Extraktion mittels des Extraktionslösungsmittels bei einer Extraktionstemperatur von unter 60 Grad Celsius durchgeführt wird, und anschliessend das Extraktionslösungsmittel bei einer Entfernungsemperatur entfernt wird, wobei die Extraktionstemperatur vorzugsweise grösser als 15 Grad Celsius und/oder unter 40 Grad Celsius gewählt ist.

18. Verfahren nach einem der Ansprüche 15 bis 17, wobei das Extraktionslösungsmittel ein wässrig-alkoholisches Lösungsmittel ist mit einem Alkoholanteil, der mehr als 0,5 Liter Alkohol pro Liter Extraktionslösungsmittel, d. h. 50 % v/v beträgt, bevorzugt mindestens 60 % v/v und besonders bevorzugt mindestens 70 % v/v.

19. Verfahren nach einem der Ansprüche 15 bis 18, wobei die Aufbereitung in der Korngrösse durch mindestens einen der folgenden Verfahrensschritte erfolgt: Vermahlen, Granulierung, Sieben, Sichten.

20. Verfahren nach einem der Ansprüche 10 bis 19, wobei das nach der Entölung erhaltene Rapsproteinmehl in der Korngrösse aufbereitet wird, um ein Schüttgut, insbesondere Pulver oder Granulat, mit einer vorgegebenen Korngrössenverteilung zu erhalten.

21. Verfahren nach einem der Ansprüche 10 bis 20, wobei die Rapssamen vor der Schälung getrocknet werden, um den Wassergehalt zu reduzieren, Enzyme zu inaktivieren und/oder eine definierte Denaturierung beim Hauptanteil des Proteins zu bewirken.

22. Verfahren nach Anspruch 21, wobei die getrockneten Rapssamen einen Wassergehalt aufweisen von weniger als 9 % w/w, bevorzugt weniger als 8 % w/w und besonders bevorzugt weniger als 7 % w/w.

23. Verwendung eines Proteinpräparats nach einem der Ansprüche 1 bis 9 zur Herstellung eines Produkts in Form eines Rapsproteinkonzentrats, eines Proteinisolates mit einem Proteingehalt von mindestens 90 % bezogen auf die Trockenmasse, eines Lebensmittels, eines Futtermittels, insbesondere Fischfutters, einer Zutat für Lebens- und/oder Futtermittel, eines Produkts für technische Anwendungen oder eines Kosmektikprodukts.

## Claims

1. Protein preparation, which is a rape protein flour produced from rape seeds, wherein the protein preparation has a protein content, which is at least 40 % and at most 70 % based on the dry mass, wherein
the protein preparation has a brightness L*, determined according to CIE-L*a*b* color scale, of at least 80 as well as at least water-binding, oil-binding and emulsifying functionality, wherein
the emulsifying capacity, determined according to the EK determination method defined in the description, is at least 600 ml/g and wherein
the protein preparation has a fat content which, based on the dry mass and determined according to Caviezel, is less than 6 %, and
a protein solubility which, determined according to the PN determination method defined in the description, is greater than 60 %.

2. Protein preparation according to claim 1, wherein the brightness L* is at least 85, preferably at least 90, and/or
the values for a* and b* according to CIE-L*a*b* color scale lie in the range of
-5 < a* < +5, -5 < b* < + 30, preferably in the range of -1 < a* < +5, 0 < b* < +25 and particularly preferred in the range of -1 < a* < +3, 0 < b* < +20.

3. Protein preparation according to any one of the preceding claims, wherein the water binding ability of the protein preparation, determined according to the AACC determination method defined in the description, is at least 1 ml per gram of dry mass, preferred at least 2 ml per gram of dry mass and particularly preferred at least 3 ml per gram of dry mass, and/or
the oil binding ability, determined according to the OB determination method defined in the description, is at least 1 ml/g, preferred at least 2 ml/g.

4. Protein preparation according to any one of the preceding claims, wherein the protein preparation has in addition foam-forming functionality.

5. Protein preparation according to claim 4, wherein the protein preparation has at least one of the following foam-forming properties:
- the foam activity corresponds to at least 30 %, preferred at least 50 % of the foam activity of hen's egg white, the foam density corresponds to at least 50 % and/or at most 200 % of the foam density of whipped egg white,
- the foam stability corresponds to at least 80 %, preferred at least 90 % and particularly preferred at least 100 % of the foam stability of whipped egg white.

6. Protein preparation according to any one of the preceding claims, wherein the protein content based on the dry mass is at least 45 % and/or at most 65 %.

7. Protein preparation according to any one of the preceding claims, wherein the protein preparation has a crude fiber content which, based on the dry mass, is at least 3 %, preferred at least 5 % and/or at most 30 %, preferred at most 20 % and particularly preferred at most 10 %.

8. Protein preparation according to any one of the preceding claims, wherein the protein preparation has a fat content which, based on the dry mass and determined according to Caviezel, is less than 5 %, preferred less than 3 %.

9. Protein preparation according to any one of the preceding claims, wherein the protein preparation has at least one content, preferred all of the following contents of substances:
- phytic acid content, based on the dry mass, of less than 10 %, preferred less than 5 %, particularly preferred less than 3 %,
- glucosinolates content of less than 4000 mg per kg of protein preparation, preferred less than 2500 mg/kg, particularly preferred less than 1500 mg/kg,
- progoitrin content of less than 1000 mg per kg of protein preparation, preferred less than 500 mg/kg, particularly preferred less than 200 mg/kg,
- phenolic acid content, based on the dry mass, of less than 5 %, preferred less than 2 %, particularly preferred less than 0.5 %.

10. Method for producing a protein preparation from rape seeds, which is a rape protein flour, comprising the following steps:
- dehulling the rape seeds so as to obtain a kernel fraction having a hull content of less than 5 % w/w,
- mechanically treating the kernel fraction, wherein the shape of the kernels is changed, and
- solvent-deoiling the mechanically treated kernel fraction by means of solvent so as to obtain the protein flour,
wherein the mechanical treatment comprises a mechanical deoiling by pressing the kernel fraction, in which a portion of the oil is separated so that the mechanically deoiled kernel fraction has a residual oil content of at least 15 % w/w and at most 35 % w/w, and wherein the temperature Tm of the kernel fraction averaged over the duration of the pressing process lies below 60 degrees Celsius.

11. Method according to claim 10, wherein the kernel fraction used for the mechanical treatment has a hull content of less than 1 % w/w.

12. Method according to any one of the claims 10 to 11, wherein the mechanical treatment comprises further a flaking of the kernels.

13. Method according to any one of the claims 10 to 12, wherein the solvent-deoiling is carried out at a first temperature and subsequently, the solvent is removed at a second temperature, wherein the first temperature lies below 90 degrees Celsius, preferred below 60 degrees Celsius, and/or wherein the second temperature lies below 90 degrees, preferred below 70 degrees Celsius.

14. Method according to any one of the claims 10 to 13, wherein the mechanically deoiled kernel fraction has a residual oil content of at most 25 % w/w, preferred at most 20 % w/w.

15. Method according to any one of the claims 10 to 14, wherein in order to obtain a protein concentrate from the rape protein flour, an extraction is carried out in which the non-protein substances are depleted from the protein flour by means of an extraction solvent and the proteins remain largely undissolved, wherein the protein concentrate obtained after the extraction is prepared with respect to the grain size so as to obtain a pourable material having a predetermined particle size distribution.

16. Method according to claim 15, wherein in the extraction an aqueous-alcoholic extraction solvent is used and the extraction is carried out in a plurality of extraction steps, wherein during at least one transition from the one to the next extraction step, the alcohol content in the extraction solvent is increased.

17. Method according to claim 15 or 16, wherein the extraction by means of the extraction solvent is carried out at an extraction temperature of less than 60 degrees Celsius and subsequently, the extraction solvent is removed at a removal temperature, wherein the extraction temperature is preferably selected to be greater than 15 degrees Celsius and/or less than 40 degrees Celsius.

18. Method according to any one of the claims 15 to 17, wherein the extraction solvent is an aqueous-alcoholic solvent which has an alcohol content of more than 0.5 liter alcohol per liter extraction solvent, i.e. 50 % v/v, preferred at least 60 % v/v and particularly preferred at least 70 % v/v.

19. Method according to any one of the claims 18 to 18, wherein preparing the grain size is carried out by at least one of the following method steps: Milling, granulating, sieving, classifying.

20. Method according to any one of the claims 10 to 19, wherein the rape protein flour obtained after deoiling is prepared with respect to the grain size so as to obtain a pourable material, in particular powder or granulate, having a predetermined grain size distribution.

21. Method according to any one of the claims 10 to 20, wherein the rape seeds are dried prior to dehulling so as to reduce the water content, inactivate enzymes and/or effect a defined denaturation in the main portion of the protein.

22. Method according to claim 21, wherein the dried rape seeds have a water content of less than 9 % w/w, preferred less than 8 % w/w and particularly preferred less than 7 % w/w.

23. Use of a protein preparation according to any one of the claims 1 to 9 for producing a product in the form of a rape protein concentrate, a protein isolate having a protein content of at least 90 % based on the dry mass, a foodstuff, a feedstuff, in particular fish food, an ingredient for foodstuff and/or feedstuff, a product for technical applications or a cosmetic product.

## Revendications

1. Préparation protéique, laquelle est une farine protéique de colza produite à partir de graines de colza, où la préparation protéique
présente une teneur en protéines, rapportée à la masse sèche, qui est égale à au moins 40 % et ne dépasse pas 70 %, où
la préparation protéique présente une clarté L*, déterminée selon la norme de mesure de couleur CIE-L*a*b*, d'au moins 80, ainsi qu'au moins une fonctionnalité liant l'eau, liant l'huile et émulsifiante, où
la capacité émulsifiante, déterminée selon le procédé de détermination EK défini dans la description, est égale à au moins 600 ml/g, et où
la préparation protéique présente une teneur en matières grasses, rapportée à la masse sèche et déterminée selon Caviezel, qui est inférieure à 6 %, et
une solubilité protéique, déterminée selon le procédé de détermination PN défini dans la description, qui est supérieure à 60 %.

2. Préparation protéique selon la revendication 1, où la clarté L* est égale à au moins 85, de préférence au moins 90, et/ou
les valeurs pour a* et b* selon la norme de mesure de couleur CIE-L*a*b* sont comprises dans la plage de -5 < a* < +5, -5 < b* < +30, de préférence dans la plage de -1 < a* < +5, 0 < b* < +25 et de manière particulièrement préférée dans la plage de -1 < a* < +3, 0 < b* < +20.

3. Préparation protéique selon l'une des revendications précédentes, où la capacité de liaison à l'eau de la préparation protéique, déterminée selon le procédé de détermination AACC défini dans la description, est égale à au moins 1 ml par gramme de la masse sèche, de préférence au moins 2 ml par gramme de la masse sèche et de manière particulièrement préférée au moins 3 ml par gramme de la masse sèche, et/ou
la capacité de liaison à l'huile, déterminée selon le procédé de détermination OB défini dans la description, est égale à au moins 1 ml/g, de préférence au moins 2 ml/g.

4. Préparation protéique selon l'une des revendications précédentes, où la préparation protéique présente en plus une fonctionnalité moussante.

5. Préparation protéique selon la revendication 4, où la préparation protéique présente l'une au moins des propriétés moussantes suivantes:
- l'activité moussante correspond à au moins 30 %, de préférence au moins 50 % de l'activité moussante du blanc d'œuf de poule,
- la densité de la mousse correspond à au moins 50 % et/ou ne dépasse pas 200 % de la densité de la mousse du blanc d'œuf de poule en neige,
- la stabilité de la mousse correspond à au moins 80 %, de préférence au moins 90 % et de manière particulièrement préférée au moins 100 % de la stabilité de la mousse du blanc d'oeuf de poule en neige.

6. Préparation protéique selon l'une des revendications précédentes, où la teneur en protéines, rapportée à la masse sèche, est égale à au moins 45 % et/ou ne dépasse pas 65 %.

7. Préparation protéique selon l'une des revendications précédentes, où la préparation protéique présente une teneur en fibres brutes, rapportée à la masse sèche, qui est égale à au moins 3 %, de préférence au moins 5 % et/ou ne dépasse pas 30 %, de préférence ne dépasse pas 20 % et de manière particulièrement préférée ne dépasse pas 10 %.

8. Préparation protéique selon l'une des revendications précédentes, où la préparation protéique présente une teneur en matières grasses, rapportée à la masse sèche et déterminée selon Caviezel, qui est inférieure à 5 %, de préférence inférieure à 3 %.

9. Préparation protéique selon l'une des revendications précédentes, où la préparation protéique présente au moins une teneur, de préférence toutes les teneurs en matières suivantes:
- teneur en acide phytique, rapportée à la masse sèche, inférieure à 10 %, de préférence inférieure à 5 %, de manière particulièrement préférée inférieure à 3 %,
- teneur en glucosinolate inférieure à 4000 mg par kg de la préparation protéique, de préférence inférieure à 2500 mg/kg, de manière particulièrement préférée inférieure à 1500 mg/kg,
- teneur en progoitrine inférieure à 1000 mg par kg de la préparation protéique, de préférence inférieure à 500 mg/kg, de manière particulièrement préférée inférieure à 200 mg/kg,
- teneur en acide phénolique, rapportée à la masse sèche, inférieure à 5 %, de préférence inférieure à 2 %, de manière particulièrement préférée inférieure à 0,5 %.

10. Procédé de production d'une préparation protéique à partir de graines de colza, laquelle est une farine protéique de colza, comprenant les étapes suivantes:
- décorticage des graines de colza pour obtenir une fraction de noyau ayant une proportion de peau de moins de 5 % w/w,
- traitement mécanique de la fraction de noyau, dans lequel la forme des noyaux est transformée, et
- déshuilage au solvant de la fraction de noyau mécaniquement traitée au moyen d'un solvant pour obtenir la farine protéique de colza,
où le traitement mécanique comprend un déshuilage mécanique par pressage de la fraction de noyau pendant lequel une partie de l'huile est séparée, de telle manière que la fraction de noyau mécaniquement déshuilée présente une teneur en huile résiduelle d'au moins 15 % w/w et ne dépassant pas 35 % w/w, et où la température moyennée sur la durée de l'opération de pressage Tm de la fraction de noyau est inférieure à 60 degrés Celsius.

11. Procédé selon la revendication 10, où la fraction de noyau utilisée dans le traitement mécanique présente une proportion de peau de moins de 1 % w/w.

12. Procédé selon l'une des revendications 10 à 11, où le traitement mécanique comprend en plus une floculation des noyaux.

13. Procédé selon l'une des revendications 10 à 12, où le déshuilage au solvant est effectué à une première température et le solvant est ensuite éliminé à une seconde température, la première température étant inférieure à 90 degrés Celsius, de préférence inférieure à 60 degrés Celsius, et/ou la seconde température est inférieure à 90 degrés Celsius, de préférence inférieure à 70 degrés Celsius.

14. Procédé selon l'une des revendications 10 à 13, où la fraction de noyau mécaniquement déshuilée présente une teneur en huile résiduelle ne dépassant pas 25 % w/w, de préférence ne dépassant pas 20 % w/w.

15. Procédé selon l'une des revendications 10 à 14, où pour obtenir un concentré protéique à partir de la farine protéique de colza est effectuée une extraction au cours de laquelle la farine de protéines est appauvrie en matières non protéiques au moyen d'un solvant d'extraction et les protéines restent largement non dissous, le concentré protéique obtenu après l'extraction étant soumis à un traitement granulométrique afin d'obtenir une matière en vrac ayant une répartition granulométrique déterminée.

16. Procédé selon la revendication 15, où lors de l'extraction est utilisé un solvant d'extraction aqueux-alcoolique et l'extraction est réalisée en plusieurs étapes d'extraction, la teneur en alcool dans le solvant d'extraction étant augmentée pendant au moins une transition d'une étape d'extraction à la suivante.

17. Procédé selon la revendication 15 ou 16, où l'extraction au moyen du solvant d'extraction est réalisée à une température d'extraction inférieure à 60 degrés Celsius et le solvant d'extraction est ensuite éliminé à une température d'élimination, la température d'extraction étant de préférence choisie supérieure à 15 degrés Celsius et/ou inférieure à 40 degrés Celsius.

18. Procédé selon l'une des revendications 15 à 17, où le solvant d'extraction est un solvant aqueux-alcoolique ayant une teneur en alcool supérieure à 0,5 litres d'alcool par litre du solvant d'extraction, c'est-à-dire égale à 50 % v/v, de préférence au moins 60 % v/v et de manière particulièrement préférée au moins 70 % v/v.

19. Procédé selon l'une des revendications 15 à 18, où le traitement granulométrique est réalisé par au moins une des étapes de procédé suivantes: broyage, granulation, tamisage, élutriation.

20. Procédé selon l'une des revendications 10 à 19, où la farine protéique de colza obtenue après le déshuilage est soumise à un traitement granulométrique afin d'obtenir une matière en vrac, plus particulièrement une poudre ou un granulat, ayant une répartition granulométrique déterminée.

21. Procédé selon l'une des revendications 10 à 20, où les graines de colza sont séchées avant le décorticage afin de réduire la teneur en eau, d'inactiver des enzymes et/ou de provoquer une dénaturation définie de la partie principale de la protéine.

22. Procédé selon la revendication 21, où les graines de colza.séchées présentent une teneur en eau inférieure à 9 % w/w, de préférence inférieure à 8 % w/w et de manière particulièrement préférée inférieure à 7 % w/w.

23. Utilisation d'une préparation protéique selon l'une des revendications 1 à 9 pour la production d'un produit sous la forme d'un concentré protéique de colza, d'un isolat de protéine ayant une teneur en protéine d'au moins 90 %, rapportée à la masse sèche, d'une denrée alimentaire, d'un aliment pour animaux, notamment d'un aliment pour poissons, d'un ingrédient pour denrées alimentaires et/ou aliments pour animaux, d'un produit destiné à des applications techniques ou d'un produit cosmétique.
